# EUROPÄISCHE PATENTANMELDUNG

(11) **EP 1 130 115 A2**
(43) Veröffentlichungstag der Anmeldung: **05.09.2001**
(21) Anmeldenummer: 01104572.1
(22) Anmeldetag: 05.03.2001
(51) Int. Cl.: C12Q 1/68

(54) **Oligonukleotidsonden zum art- und/oder gattungsspezifischen Nachweis von das Pflanzenwachstum fördernden Bakterien**

(30) Priorität: 03.03.2000 DE 10010614
(71) Anmelder: GSF - Forschungszentrum für Umwelt und Gesundheit, GmbH, 85758 Oberschleissheim (DE)
(72) Erfinder: Stoffels, Marion, Dr., 81539 München (DE); Hartmann, Anton, Prof. Dr., 85716 Unterschleissheim (DE); Munch, Jean Charles, Prof. Dr., 73760 Ostfildern (DE)
(74) Vertreter: Maiwald Patentanwalts GmbH

(57) **Zusammenfassung**

Die Erfindung betrifft Oligonukleotidsonden zum art- und/oder gattungsspezifischen Nachweis von das Pflanzenwachstum fördernden Bakterien (***p**lant **g**rowth **p**romoting bacteria*, PGPR-Bakterien), sowie Verfahren zum art- und/oder gattungsspezifischen Nachweis dieser Bakterien.

## Beschreibung

Die Erfindung betrifft Oligonukleotidsonden zum art- und/oder gattungsspezifischen Nachweis von das Pflanzenwachstum fördernden Bakterien (***p**lant **g**rowth **pr**omoting bacteria*, PGPR-Bakterien), sowie Verfahren zum art- und/oder gattungsspezifischen Nachweis dieser Bakterien.

Ein optimales Wachstum sowie damit verbunden ein hoher Ertrag und die gute Gesundheit von Kulturpflanzen sind mit einer guten Nährstoffversorgung und der Kontrolle von phytopathogenen Keimen verbunden. Die starke Düngung von Kulturpflanzen mit den Nährelementen Stickstoff, Phosphat, Kalium, Magnesium und dergleichen führt zu gravierenden Umweltproblemen, da mehr oder weniger große Teile der Düngungsmengen nicht genutzt werden und somit benachbarte Umweltkompartimente wie Wasser und Luft belastet werden.

Die Pflanzenernährung und die Gesundheit der Pflanzen können jedoch unter Beteiligung von *plant growth promoting bacteria* (PGPR-Bakterien) oder durch Unterdrückung von phytopathogenen Mikroorganismen im Wurzelraum, die das Wachstum der Pflanze beeinträchtigen oder sogar schwer schädigen, verbessert werden. Sogenannte *plant growth promoting bacteria* besiedeln das Wurzelsystem von Pflanzen und fördern über verschiedene Mechanismen das Pflanzenwachstum (Y. Okon und C.A. Labandera-Gonzalez (1994), Soil Biol. Biochem. 26, 1591-1601; A. Hartmann (1996), "Biotechnological aspects of diazotrophic bacteria associated with rice" in: Biological nitrogen fixation associated with rice production (Hrsg.: M. Rahman et al.), Kluwer Academic Publishers, Dordrecht, Niederlande, 211-223). Einer dieser Mechanismen ist die bakterielle Produktion von verschiedenen Phytohormonen, die die Wurzelentwicklung stimulieren und damit die Aufnahme von Nährstoffen und Wasser durch die Pflanze fördern. Dadurch können die erforderlichen Düngemengen verringert werden, wodurch auch die potentiell umweltbelastenden Düngeverluste reduziert werden können.

Des weiteren können bestimmte, mit der Wurzel assoziierte oder endophytisch vorkommende diazotrophe Bakterien atmosphärischen Stickstoff zu pflanzenverwertbarem Düngerstickstoff Ammonium reduzieren. Der Anteil von derart biologisch fixiertem Stickstoff am Gesamt-Stickstoff ist bei verschiedenen Nicht-Leguminosen sehr unterschiedlich: Bei einigen Zuckerrohrsorten kann er bis zu 40-60% betragen, dies entspricht etwa 150 kg Stickstoff pro Hektar und Jahr (R.M. Boddey et al. (1995), Plant Soil 174, 195-209). Bei Reis ist dagegen der Anteil biologischer Stickstoffixierung am Pflanzenstickstoff mit 10 bis 60 kg Stickstoff pro Hektar und Jahr wesentlich niedriger.

Als diazotrophe PGPR-Bakterien sind u.a. eine Reihe Gram-negativer Bakterien der alpha- und beta-Untergruppe der Proteobakterien wie die Gattungen *Azospirillum*, *Acetobacter* und *Herbaspirillum* bekannt, welche die Wurzelsysteme verschiedenster bedeutender Kulturpflanzen, beispielsweise Weizen, Reis, Zuckerrohr und Mais und dergleichen, besiedeln. Diese Bakterien besiedeln die Wurzeloberfläche oder sind als endophytische Populationen in den Wurzeln und auch in Stengeln und Blättern der Pflanzen vorhanden (J. Döbereiner (1995), "Isolation and identification of aerobic nitrogen-fixing bacteria from soil and plants" in: Methods in Applied Soil Microbiology and Biochemistry (Hrsg.: K. Alef und P. Nannipieri), Academic Press, London, 134-141; J. Döbereiner et al. (1993), "Endophytic diazotrophs in sugar cane, cereals and tuber plants" in: New horizons in nitrogen fixation (Hrsg.: R. Palacios et al.), Kluwer Academic Publishers, Dordrecht, Niederlande, 671-676). *Acetobacter diazotrophicus* liegt bei Zuckerrohr vorzugsweise als endophytisches Bakterium vor (E. K. James und F. L. Olivares (1998), Crit. Rev. Plant Sci. 17, 77-119), während *Herbaspirillum* spp. eine Vielzahl von Gramineen u.a. auch endophytisch besiedeln (F. L. Olivares et al. (1996) Biol. Fertil. Soils 21, 197-200; G. Kirchhof et al. (1997), Soil Biol. Biochem. 29, 853-862).

Es gibt derzeit verschiedene biotechnologische Entwicklungen, diazotrophe PGPR-Bakterien in bzw. an den Pflanzen durch unterschiedliche Inokulationsstrategien zu etablieren (V. M. Reis (1999), Plant Soil 206, 205-211). Es sind jedoch bislang keine einfachen und zuverlässigen Methoden zum spezifischen Nachweis des Erfolges dieser Inokulationen und des Vorkommens von aktiven PGPR-Bakterien in diesen Pflanzen in Abhängigkeit beispielsweise von der Bewirtschaftung bekannt. Ein derartiger Nachweis ist jedoch äußerst wünschenswert, um das biologische Potential dieser Bakterien zielgerichtet und zuverlässig zu entwickeln und wirtschaftlich auszunutzen.

Bislang sind eine Reihe von Identifizierungs- und Quantifizierungsmethoden für PGPR-Bakterien bekannt, die jedoch für eine Lösung der vorstehenden Probleme ungeeignet sind. So werden bei der MPN-Methode (most probable number-Methode, Döbereiner, 1995, supra) diazotrophe Bakterien der Gattungen *Azospirillum*, *Herbaspirillum* und *Acetobacter* in verschiedenen halbselektiven, semisoliden stickstofffreien Nährmedien kultiviert. Für die Erfassung der Anzahl lebensfähiger Bakterien in einer bestimmten Probe durch Kultivierung werden Medien bzw. Bedingungen gewählt, die eine selektive Erfassung bestimmter Gruppen ermöglichen. So wurden beispielsweise von bakteriellen Einzelkolonien Reinkulturen angelegt und diese aufgrund phänotypischer Merkmale wie ihrer Morphologie und ihrer Stoffwechselwege identifiziert. Die Identifizierung von Bakterien nach vorheriger Kultivierung ist jedoch mit entscheidenden Nachteilen verbunden. So ist mit dieser Kultivierungstechnik keine Identifizierung auf Gattungs- oder Artebene möglich, da die verwendeten Medien nicht die entsprechende Selektivität aufweisen. Ferner belegen Untersuchungen an verschiedensten Umweltproben, daß derzeit nur 0,1 bis 14% aller Bakterien kultivierbar sind. Des weiteren ist die Methode sehr zeitaufwendig, da mehrtägige Inkubationen erforderlich sind. Schließlich treten bei der Kultivierung starke Populationsverschiebungen auf, d.h. bestimmte Bakteriengruppen werden durch die gewählten Kultivierungsbedingungen im Labor bevorzugt und andere werden diskriminiert.

Ferner sind serologische Verfahren zur Analyse von PGPR-Bakterien bekannt. So sind für einige dieser Bakterien poly- und monoklonale Antikörper beschrieben (M. Schloter et al. (1997), Hybridoma 16, 183-187; G. Kirchhof et al. (1997), Soil Biol. Biochem. 29, 853-862), die eine spezifische Identifizierung und Quantifizierung von Teilen dieser Bakterienpopulationen im ELISA-Test erlauben (M. Schloter et al. (1992), Soil Biol. Biochem. 24, 399-403). Polyklonale Antiseren, d.h. Gemische aus verschiedenen Antikörpern mit unterschiedlicher Spezifität, weisen jedoch aufgrund ihrer eingeschränkten Spezifität und auch aufgrund ihrer eingeschränkten Sensitivität prinzipielle Nachteile auf. Ferner ist durch das Vorhandensein von verschiedenen Antikörpern eine Validierung von quantitativen Testsystemen nicht möglich. Die für einige *Azospirillum brasilense*-Stämme beschriebenen monoklonalen Antikörper (Kirchhof et al., supra; Schloter et al., supra) sind stammspezifisch und kommen daher für eine breitere Anwendung zum Nachweis von PGPR-Bakterien nicht in Frage.

Schließlich wurden im Stand der Technik Oligonukleotidsonden beschrieben, die gegen die rRNA (ribosomale RNA) von PGPR-Bakterien gerichtet sind. Nachweisverfahren unter Verwendung dieser Sonden basieren auf der Tatsache, daß die in jeder Zelle vorhandenen rRNAs hochkonservierte und variable Sequenzabschnitte umfassen. Deshalb können sie als Zielmoleküle für diagnostische Sonden auf unterschiedlichen phylogenetischen Ebenen genutzt werden. Bereits Mitte der 80er Jahre wurde gezeigt, daß die Sequenzen der 16S- und 23S-rRNA zur Identifizierung von Mikroorganismen genutzt werden können (Woese (1987), Microbiol. Reviews 51, 221; De Long et al. (1989), Science 243, 1360).

Beispielsweise sind für einige der PGPR-Bakterien phylogenetische Oligonukleotidsonden zur Identifizierung bekannt (G. Kirchhof and Hartmann (1992) Symbiosis 13, 27-35; M.M. Kabir et al. (1995), Can. J. Microbiol. 41, 1081-1087), die vor allem in dot blot-Analysen und PCR-Amplifikationen (Kirchhof et al., supra) angewandt werden. Mit diesen 23S- und 16S-rRNA-gerichteten Oligonukleotidsonden ist jedoch keine bzw. nur eine schwache in situ-Einzelzellhybridisierung möglich, weshalb diese Oligonukleotidsonden nur für die methodisch sehr aufwendige dot blot-Hybridisierung mit isolierten Nukleinsäuren eingesetzt werden können. Ferner weisen die Oligonukleotidsonden von Kabir et al. (supra) beträchtliche Unspezifitäten auf, so daß eine Anwendung dieser Sonden für eine sichere Identifizierung von PGPR-Bakterien nicht empfehlenswert ist. Die für *Herbaspirillum* beschriebenen Oligonukleotidsonden (J. I. Baldani et al. (1996), Int. J. System. Bacteriol. 46, 802-810) sind ebenfalls nicht für eine in situ-Einzelzellhybridisierung geeignet und außerdem in ihrer Spezifität nicht optimal. Ebenso sind die bisher beschriebenen *Acetobacter diazotrophicus*-Sonden für eine Ganzzellhybridisierung nur schlecht einsetzbar.

Angesichts der vorstehend beschriebenen Nachteile der bisher bekannten Verfahren zum Nachweis von PGPR-Bakterien ist es eine wesentliche Aufgabe der Erfindung, Sonden bereitzustellen, mittels denen bzw. auf deren Grundlage PGPR-Bakterien in Pflanzen oder in Pflanzenextrakten in situ spezifisch, einfach und zuverlässig nachgewiesen werden können. Des weiteren ist es wünschenswert, Nachweisverfahren bereitzustellen, um das natürliche Vorkommen von PGPR-Bakterien in unterschiedlichen Kultivaren und Zuchtlinien von Pflanzen zu untersuchen. Weitere Aufgaben ergeben sich aus der folgenden Beschreibung.

Diese Aufgaben werden durch die Gegenstände der unabhängigen Ansprüche, insbesondere basierend auf der Bereitstellung der erfindungsgemäßen Oligonukleotidsonden zum art- und/oder gattungsspezifischen Nachweis von PGPR-Bakterien, insbesondere von Bakterien der Gattungen *Azospirillum* und *Herbaspirillum* sowie für die Art *Acetobacter diazetrophicus*, gelöst.

Vorteilhafte Ausgestaltungen sind in den Unteransprüchen beschrieben.

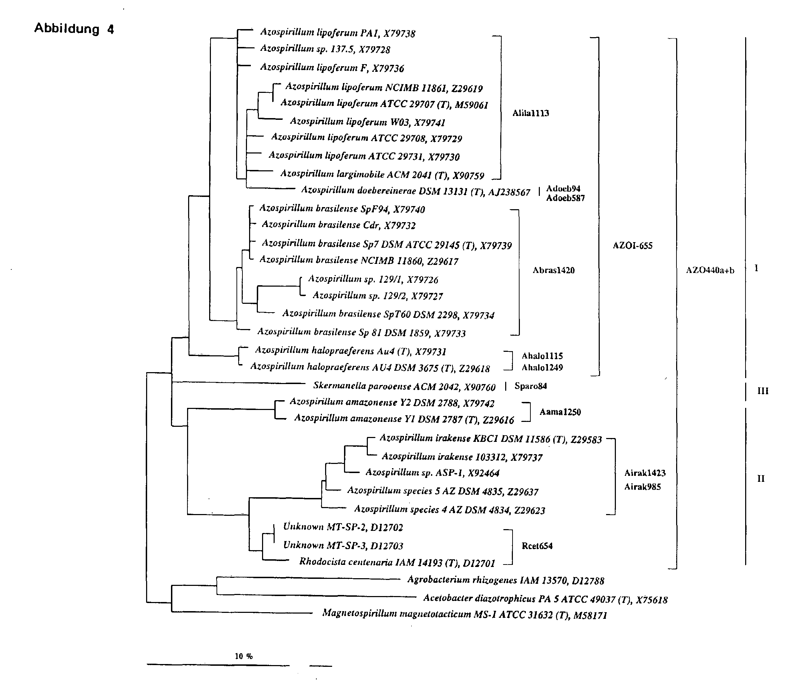

Die Erfindung basiert auf der Bereitstellung von Oligonukleotidsonden zum art- und/oder gattungsspezifischen Nachweis von PGPR-Bakterien, wobei die Oligonukleotidsonden über in situ-Einzelzellhybridisierung an die 16S-rRNA in intakten Bakterienzellen binden. Es werden ein geschachtelter Sondensatz für das *Azospirillum-Rhodocista-Skermanella-Cluster* und die einzelnen Arten/Gattungen dieses Clusters (vgl. Abb. 4) sowie ein ebenfalls geschachtelter Sondensatz für die Gattung *Herbaspirillum* und die dazugehörenden Arten bereitgestellt. Ferner wird für die Art *Acetobacter diazotrophicus* eine Oligonukleotidsonde bereitgestellt.

Insbesondere werden als erfindungsgemäße Oligonukleotidsonden die in SEQ:ID Nr. 1-18 gezeigten Sequenzen für das Gattungscluster *Azospirillum*, *Skermanella*, *Rhodocista*, die in SEQ:ID Nr. 19-23 gezeigten Sequenzen für die Gattung *Herbaspirillum*, sowie die in SEQ:ID

Nr. 24 gezeigte Sequenz für *Acetobacter diazotrophicus* bereitgestellt Ferner umfasst die vorliegende Erfindung auch Nukleinsäuremoleküle, die die in SEQ:ID Nr. 1-18 aufgeführten Oligonukleotidsonden oder Fragmente davon umfassen und durch in situ-Einzelzellhybridisierung an die 16S-rRNA der vorstehend genannten Arten bzw. Gattungen spezifisch binden. Auch von der Erfindung umfaßt sind solche DNA-Sequenzen, die sich von den in SEQ:ID Nr. 1-18 aufgeführten Sonden in einzelnen oder mehreren Nukleotiden, vorzugsweise in bis zu drei Basen unterscheiden, sowie Nukleinsäuremoleküle, die diese DNA-Sequenzen umfassen und Fragmente davon, wobei erfindungsgemäß eine spezifische in situ-Einzelzellhybridisierung an die 16S-rRNA der vorstehend genannten Arten bzw. Gattungen gewährleistet ist.

Des weiteren wird durch die vorliegende Erfindung ein Verfahren zum art- und/oder gattungsspezifischen Nachweis von PGPR-Bakterien durch in situ-Einzelzellhybridisierung bereitgestellt. Das erfindungsgemäße Verfahren umfaßt vorzugsweise die folgenden Schritte:
a) Fixieren der PGPR-Bakterien;
b) In situ-Inkubation der fixierten Bakterien mit Oligonukleotidsonden, die spezifisch an die 16S-rRNA von PGPR-Bakterienzellen hybridisieren; und
c) Detektieren und gegebenenfalls Quantifizieren der PGPR-Bakterien.

Die Entwicklung eines kompletten Satzes der erfindungsgemäßen diagnostischen Oligonukleotidsonden für die Identifizierung, Detektion und Quantifizierung wichtiger PGPR-Bakterien mittels in situ-Einzelzellhybridisierung erlaubt eine schnelle und einfache Identifizierung auf unterschiedlichen phylogenetischen Ebenen. Die erfindungsgemäßen Oligonukleotidsonden sind so entwickelt, daß sie an die 16S-rRNA in Bakterien spezifisch binden, die vor der Inkubation mit den erfindungsgemäßen Oligonukleotidsonden fixiert wurden. Somit ist bei dem erfindungsgemäßen Verfahren zum art- und/oder gattungsspezifischen Nachweis von PGPR-Bakterien weder eine aufwendige Nukleinsäureisolierung aus bakteriellen Reinkulturen oder komplexen Umweltproben mit anschließender dot blot-Hybridisierung noch ein aufwendiger Kultivierungsschritt notwendig.

Die erfindungsgemäßen Oligonukleotidsonden weisen ferner den besonderen Vorteil auf, daß es sich hierbei um einen genau aufeinander abgestimmten Sondensatz für die jeweiligen Gattungen handelt, der eine zuverlässige Identifizierung der jeweiligen PGPR-Bakterien gewährleistet. Dadurch, daß die Zielregionen der neuen Sonden auf der 16S-rRNA für in situ-Einzelzellhybridisierung geeignet sind, ist die Möglichkeit einer direkten Identifizierung sowie einer Lokalisierung und Quantifizierung der Bakterien im Pflanzenmaterial gegeben.

In situ ist im Zusammenhang der vorliegenden Erfindung dahingehend zu verstehen, daß die zu untersuchenden Bakterienzellen noch intakt sind und molekularbiologisch identifiziert werden können. Durch die vorstehend erwähnte Fixierung der Zellen bleibt die Morphologie der Bakterien erhalten. Die Förderung des Pflanzenwachstums kann daher mittels der erfindungsgemäßen Oligonukleotidsonden auf bestimmte Bakterien bezogen werden, wodurch im Unterschied zu den Verfahren des Standes der Technik PGPR-Bakterien auf unterschiedlichen phylogenetischen Ebenen identifiziert und gegebenenfalls in Pflanzenmaterial lokalisiert und quantifiziert werden können.

Im Rahmen der vorliegenden Erfindung soll unter Fixieren von Bakterien eine Behandlung verstanden werden, mit der die den jeweiligen Mikroorganismus umgebenden Hüllen so durchlässig gemacht werden sollen, daß die Oligonukleotidsonde mit einem gegebenenfalls kovalent verbundenen detektierbaren Marker durch die Zellwand des Bakteriums penetrieren kann, um so die Zielsequenzen im Zellinneren erreichen zu können. Zur Fixierung wird üblicherweise eine geringprozentige Paraformaldehydlösung verwendet. Sollte mit einer Paraformaldehydlösung im Einzelfall die Bakterienzellwand nicht penetrierbar gemacht werden können, so sind dem Fachmann ausreichend weitere Maßnahmen bekannt, die zu demselben Ergebnis führen. Dazu zählen beispielsweise Ethanol, Methanol, Mischungen dieser Alkohole mit Paraformaldehyd, Glutaraldehyd, enzymatische Behandlungen, Ultraschallbehandlung usw.

Erfindungsgemäß erfolgt die Detektion der erfindungsgemäßen Oligonukleotidsonden über an die Oligonukleotidsonden gekoppelte detektierbare Marker oder über mit detektierbaren Markern gekoppelte Antikörper. Als detektierbare Marker können bspw. fluoreszierende Gruppen eingesetzt werden, wie FITC (erhältlich von Sigma Aldrich, Deisenhofen, Deutschland), TRITC (Tetramethylrhodamin-5,6-isothiocyanat, erhältlich von Sigma Aldrich), CY-3 (NHS-Ester von Cy5.18, erhältlich von Molecular Probes, Leiden, Niederlanden), CY-5 (NHS-Ester von Cy5.18, erhältlich von Molecular Probes, Leiden, Niederlanden), die dem Fachmann wohl bekannt sind. Alternativ können auch andere Marker wie chemolumineszierende Gruppen oder radioaktive Markierungen eingesetzt werden. Auch eine DIG- oder Biotin-Markierung der Oligonukleotidsonden bietet sich an. Weiter können die Oligonukleotidsonden je nach verwendeter Markierung auch durch enzymatisch aktive Moleküle nachgewiesen werden. Dabei können z.B. Enzyme wie alkalische Phosphatase, saure Phosphatase, Peroxidasen, β-D-Galaktosidase oder Glukoseoxidase als Marker eingesetzt werden. Auch hier sind dem Fachmann geeignete Enzyme und entsprechende Chromogene bekannt und kommerziell erhältlich.

Bei einer erfindungsgemäßen Oligonukleotidsonde kann es sich um eine DNA- oder RNA-Sonde handeln, die in der Regel zwischen 10 und 100, bevorzugt zwischen 12 und 50 und besonders bevorzugt zwischen 15 und 20 Nukleotide umfassen wird. Die Oligonukleotidsonde ist so ausgewählt, daß es eine zu ihr komplementäre Sequenz in der Art bzw. der Gattung der nachzuweisenden PGPR-Bakterien gibt. Komplementarität bei einer Sonde mit etwa 15 bis 20 Nukleotiden liegt vor, wenn ein oder zwei, vorzugsweise aber keine Fehlpaarungsstelle vorliegt, mit der Maßgabe, daß die Oligonukleotidsonde unter stringenten Hybridisierungsbedingungen mit der Zielsequenz des PGPR-Bakteriums, d.h. einer Sequenz der 16S-rRNA, tatsächlich hybridisiert. Stringente Bedingungen im Sinne der Erfindung sind beispielsweise eine Formamidkonzentration von 15 bis 60% in einem herkömmlichen Hybridisierungspuffer.

Die Hybridisierungsdauer sollte üblicherweise mindestens 1,5 h betragen, in der Regel beträgt sie 1,5 bis 2 h. Hybridisierungen von Pflanzenmaterial erfolgen teilweise auch über Nacht. Allgemein sollte die Hybridisierung in feuchter Kammer durchgeführt werden; die Hybridisierungstemperatur liegt in der Regel bei 46°C.

Ein typischer Hybridisierungspuffer hat folgende Zusammensetzung: 0,9 M NaCl, 20 mM Tris-HCl (pH 8,0), 0,01% SDS und 0-65% Formamid, je nach den Stringenzbedingungen der eingesetzten Sonde.

Die Konzentration der jeweiligen Oligonukleotidsonde sollte 20-50 ng je 8 µl Hybridisierungspuffer betragen.

Der Waschpuffer hat herkömmlicherweise folgende Zusammensetzung: NaCl, 20 mM Tris-HCl, 0,01% SDS. Um größere Abfallmengen an Formamid zu vermeiden, wird im Waschpuffer in der Regel die identische Stringenz wie im Hybridisierungspuffer durch eine Absenkung der Kationen-Konzentration eingestellt. Die hierfür benötigte NaCl-Konzentration kann nach der in Wetmur J. (1991) DNA Probes: applications of the principle of nucleic acid hybridization. Crit. Rev. Biochem. Mol. Biol. 26:227-259 bzw. Wahl et al. (1987) Molecular hybridization of immobilized nucleic acids: theoretical concepts and practical considerations. Methods in Enzymology 152:399-407, in diesem Zusammenhang angebenen Formel berechnet werden. Bei geringen NaCl-Konzentrationen ist eine Zugabe von EDTA-Lösung, z.B. Na₂EDTA-Lösung, bis zu einer Endkonzentration von 5 mM im Waschpuffer nützlich, da ansonsten bereits Spuren zweiwertiger Kationen durch ihre starke Hybridstabilisierung die Stringenz beeinflussen können.

Nach der Hybridisierung werden die Proben vorzugsweise mit vorgewärmtem Waschpuffer (48°C) abgespült und dann beispielsweise für 15 bis 20 Minuten im vorgewärmten Waschpuffer bei 48°C inkubiert. Anschließend werden sie mit destilliertem Wasser abgespült und an der Luft getrocknet.

Doppelhybridisierungen mit Oligonukleotidsonden, welche unterschiedliche Formamidkonzentrationen zur Einhaltung der Stringenz benötigen, werden üblicherweise sukzessive durchgeführt. Hierbei erfolgt zuerst die Hybridisierungsprozedur mit der Sonde, die eine höhere Formamidkonzentration benötigt. Nach Beendigung der Waschphase und anschließender Lufttrocknung kann eine zweite Hybridisierungsprozedur, beginnend bei der Zugabe der Hybridisierungslösung, angeschlossen werden.

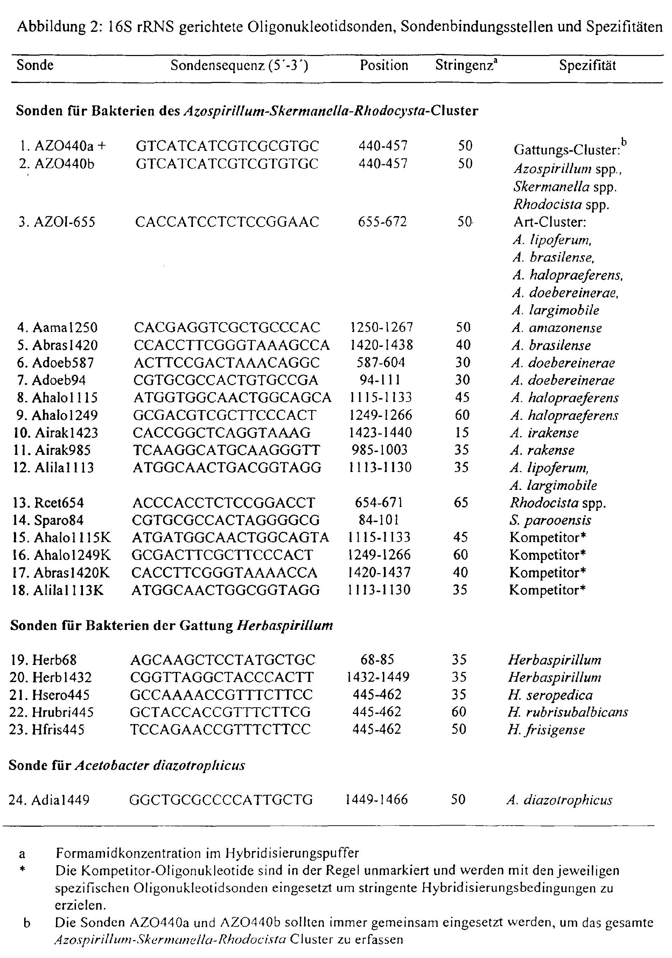

Die Spezifität der Sonden wurde durch Einzelzellhybridisierung mit fluoreszenzmarkierten Sonden und repräsentativen Referenzorganismen getestet. Die Stringenz der Hybridisierungen wurde schrittweise durch die Zugabe von Formamid in 5%-Schritten zum Hybridisierungspuffer erhöht. Die Formamid-Konzentrationen, die in beigefügter Abbildung 2 als optimal empfohlen werden, sind die höchsten, die noch gute Signale mit den Zielzellen bei einer gleichzeitigen Diskriminierung der Nicht-Zielzellen ermöglichen.

Einige der neu entwickelten artspezifischen Oligonukleotidsonden für die Azospirillen zeigten nicht die gewünschte Spezifität (z.B. Ahalo 1115, Ahalo 1249, Abras 1420 und Alila 1113). Die Spezifität der Hybridisierung konnte allerdings nicht durch eine weitere Erhöhung der Formamidkonzentration im Hybridisierungspuffer erhöht werden ohne einen signifikanten Verlust der Signalintensität hinnehmen zu müssen. Deshalb wurden Kompetitor-Oligonukleotidsonden entwickelt (Ahalo 1115K, Ahalo 1249K, Abras 1420K, Alila 1113K). Kompetitor-Oligonukleotidsonden bieten die Möglichkeit die Spezifität zu erhöhen, ohne die Sensitivität negativ zu beeinflussen (Manz et al. (1992) System. Appl. Microbiol. 15, 593-600).

Die Auswahl der jeweiligen Oligonukleotidsonden erfolgt in Abhängigkeit der nachzuweisenden PGPR-Bakterien. Sollen beispielsweise nur PGPR-Bakterien der Art *Herbaspirillum seropedica* nachgeweisen werden, nicht jedoch PGPR-Bakterien der Art *Herbaspirillum rubrisubalbicans*, so wird der Fachmann eine geeignete Sequenz auswählen, die in *Herbaspirillum seropedica* auftritt, in *Herbaspirillum rubrisubalbicans* und allen anderen Bakterien, für die 16S- und 23S-rRNA-Sequenzinformationen zur Verfügung stehen, dagegen nicht.

Die in SEQ:ID Nr. 1-24 gezeigten Sequenzen oder Fragmente davon beziehungsweise Nukleinsäuremoleküle, die die vorstehend beschriebenen erfindungsgemäßen Oligonukleotidsonden oder Teile davon umfassen, stellen nützliche Hybridisierungssonden für PGPR-Bakterien des Gattungsclusters *Azospirillum*, *Skermanella*, *Rhodocista*, sowie der Gattungen *Herbaspirillum* dar, da sie mit der 16S-rRNA dieser Bakterien spezifisch reagieren.

Ferner werden gemäß der vorliegenden Erfindung auf der Grundlage der in SEQ:ID Nr. 1-24 gezeigten DNA-Sequenzen konstruierte Oligonukleotidprimer und Paare von Oligonukleotidprimern bereitgestellt, die einen art- und/oder gattungsspezifischen Nachweis und eine Quantifizierung der 16S-rRNA-Nukleinsäuren von PGPR-Bakterien mittels PCR-Technik ermöglichen. Dabei können die erfindungsgemäßen 16S-rRNA-spezifischen Primer auch zusammen mit anderen Primern eingesetzt werden, beispielsweise solchen Primern, die von einer konservierten Sequenzregion innerhalb der 16S- oder 23S-rRNA abgeleitet worden sind.

Die Erfindung betrifft auch Fragmente der in SEQ:ID Nr. 1-24 gezeigten Sequenzen, die eine ausreichende Länge aufweisen, um mit der 16S-rRNA von PGPR-Bakterien spezifisch hybridisieren zu können. Die erfindungsgemäßen Oligonukleotidsonden, die einen speziesspezifischen Nachweis und eine speziesspezifische Quantifizierung von PGPR-Bakterien ermöglichen, umfassen solche Oligonukleotide, deren Sequenz in einzelnen oder mehreren Nukleotiden durch Addition, Deletion, Substitution oder chemische Modifikationen von den in SEQ:ID Nr. 1-24 gezeigten Sequenzen unmittelbar abgeleiteten Oligonukleotid-Sequenzen abweichen, soweit gleichzeitig eine spezifische Hybridisierung solcher Sonden an die 16S-rRNA von PGPR-Bakterien gewährleistet ist. Gleiches gilt selbstverständlich für die als Hybridisierungssonde eingesetzten Fragmente der in SEQ:ID Nr. 1-24 gezeigten Sequenzen.

Weiter umfaßt die Erfindung auch solche DNA-Sequenzen, deren Nukleotidsequenz zu den erfindungsgemäßen DNA-Sequenzen komplementär ist oder die den von den erfindungsgemäßen DNA-Sequenzen abgeleiteten RNA-Sequenzen entsprechen. Ebenfalls sind Gegenstand der Endung solche DNA-Sequenzen, die zu den erfindungsgemäßen DNA-Sequenzen eine Sequenzhomologie von mindestens 80%, 85%,vorzugsweise von mindestens 90%, 92% und besonders bevorzugt von mindestens 95%, 97% aufweisen.

Ferner umfaßt die Erfindung solche DNA-Sequenzen, die mit der 16S-rRNA von PGPR-Bakterien unter üblichen Hybridisierungsbedingungen, vorzugsweise unter stringenten Hybridisierungsbedingungen, hybridisieren. Generell umfaßt die Erfindung solche DNA-Sequenzen, die eine art- und/oder gattungsspezifische Hybridisierung mit den und/oder eine Amplifizierung der erfindungsgemäßen Oligonukleotidsonden ermöglichen. Der Fachmann, der mit gängigen Hybridisierungs- bzw. Amplifizierungsmethoden vertraut ist, ist ohne Schwierigkeiten in der Lage zu erkennen, ob eine art- und/oder gattungsspezifische Bindung der jeweiligen Oligonukleotidsonde bzw. des jeweiligen Oligonukleotidprimers mit der 16S-rRNA von PGPR-Bakterien gegeben ist, oder ob es sich gegebenenfalls um eine unspezifische, von der Erfindung nicht umfaßte Bindung handelt. Während im Fall der PCR bei einer unspezifischen Amplifikation unerwünscht zusätzliche Reaktionsprodukte auftreten bzw. in der Gelelektrophorese ein diffuser Nukleinsäureschmier zu beobachten ist, haben im Rahmen von Hybridisierungsreaktionen unspezifische Wechselwirkungen zur Folge, daß sich die Hybridisierungssonde bei hohen Temperaturen und geringen Salzkonzentrationen (beispielsweise 0,1 x SSC und 0,5% SDS) vom Target ablöst. Andererseits werden bei zu geringer Stringenz der Hybridisierung auch Nicht-Zielzellen mit den Oligonukleotidsonden detektiert.

Neben dem erfindungsgemäßen Nachweis-Verfahren durch situ-Einzelzellhybridisierung können die erfindungsgemäßen Oligonukleotidsonden und Oligonukleotidprimer-Sequenzen auch in herkömmlichen Hybridisierungsverfahren wie beispielsweise Dot-Blot-Hybridisierungen als auch in Amplifikationsverfahren, insbesondere in diagnostischen Polymerase-Ketten-Reaktionen (*polymerase chain reaction*, PCR) zum art- und gattungsspezifischen Nachweis von PGPR-Bakterien eingesetzt werden. Ferner sind die erfindungsgemäßen Oligonukleotidsonden für einen Einsatz bei einer Miniaturisierung eines der vorstehend beschriebenen Nachweisverfahrens auf Mikrochips geeignet. Dem Fachmann sind die grundlegenden Methoden bekannt; sie sind auch in gängigen molekularbiologischen Laborhandbüchern beschrieben, wie beispielsweise in Sambrook et al. (1989), Molecular Cloning: A Laboratory Manual, 2. Aufl., Cold Spring Harbour Laboratory Press, Cold Spring Harbour, New York.; Griffin und Griffin (1994), PCR-Technology - Current Innovations, CRC Press, Boca Raton, Ann Arbor, London, Tokyo.

Bei einer besonderen Ausführungsform der vorliegenden Erfindung umfaßt das Verfahren zum art- und/oder gattungsspezifischen Nachweis von das Pflanzenwachstum fördernden Bakterien die folgenden Schritte:
a) Fixieren der PGPR-Bakterien in/auf Pflanzenmaterial, in Reinkulturen (Isolaten) oder in Anreicherungskulturen;
b) Inkubation der fixierten Proben mit den erfindungsgemäßen mit einem Fluoreszenzmarker gekoppelten Oligonukleotidsonden;
c) Detektieren und gegebenenfalls Quantifizieren der markierten PGPR-Bakterien.

Optional erfolgt zwischen Schritt b) und c) in einem Schritt b1) ein Abwaschen der nicht gebundenen Sondenmoleküle.

Bei dem erfindungsgemäßen Verfahren zum art- und/oder gattungsspezifischen Nachweis von Bakterien werden die erfindungsgemäßen Oligonukleotidsonden bei der in situ-Zellhybridisierung vorzugsweise mit Fluoreszenzmarkern eingesetzt, so daß eine einfache Detektion mittels Fluoreszenzmikroskopie möglich ist. Eine alternative Ausführungsform der vorliegenden Erfindung ist eine Markierung durch Biotin- oder Digoxigeninkopplung. Die biotinylierten Sonden werden anschließend über Streptavidin-gekoppelte Konjugate, die Digoxigenin-markierten Sonden über Antikörper detektiert.

Die Erfindung betrifft des weiteren einen Kit, der für den art- und/oder gattungsspezifischen Nachweis von PGPR-Bakterien eingesetzt werden kann und eine oder mehrere erfindungsgemäße Oligonukleotidsonden bzw. Nukleinsäuremoleküle umfaßt. Der bzw. die Oligonukleotidsonden können beispielsweise in lyophilisierter Form oder in einem geeigneten Puffer vorliegen. Weitere Kit-Bestandteile können Enzyme und Reagenzien sein, die üblicherweise bei Hybridisierungs- oder PCR-Reaktionen eingesetzt werden, die ebenfalls in lyophilisierter Form oder in geeigneten Puffern vorliegen. Der Kit kann selbstverständlich auch sämtliche zusätzliche, für das erfindungsgemäße Nachweisverfahren erforderlichen Elemente umfassen.

Die vorliegende Erfindung betrifft ebenfalls die Verwendung der erfindungsgemäßen Oligonukleotidsonden bzw. Nukleinsäuremoleküle zum Nachweis von das Pflanzenwachstum fördernden Bakterien. Insbesondere können die erfindungsgemäßen Oligonukleotidsonden zur Lokalisierung und Quantifizierung von PGPR-Bakterien in Pflanzenmaterialien, aber alternativ auch zur Identifizierung von Bakterienisolaten oder in automatisierten Testverfahren, auch auf Mikrochips, beispielsweise in Kombination mit Biotin- und Digoxigeninkopplung und nachfolgender Antikörperdetektion, verwendet werden.

Die nachfolgenden Beispiele dienen der Erläuterung der Erfindung, ohne diese in irgendeiner Weise einzuschränken.

### Beispiele

### Beispiel 1: Oligonukleotidsonden für den artspezifischen Nachweis von Azospirillum

### Isolierung der Azospirillum-Stämme:

Wurzeln von *Miscanthus sinensis* cv Giganteus und Rhizosphären-Erde wurden von Feldern der LBP (Bayerische Landesanstalt für Bodenkultur und Pflanzenbau), Freising, Deutschland gesammelt, auf denen seit 1990 kontinuierlich Miscanthus kultiviert wurde. Die Wurzeln wurden mit sterilem Wasser gewaschen und dann in 4%iger Saccharose-Lösung unter Verwendung von Mörser und Stößel zerkleinert. Kleine Gefäße (etwa 10 ml), die 5 ml an semisolidem, stickstofffreiem NFb-Medium enthalten (Döbereiner (1995), supra), wurden mit Verdünnungen der zerkleinerten Wurzeln bzw. Rizosphären-Erde-Suspensionen inokuliert. Die Zusammensetzung des NFb-Mediums war (Mengenangaben pro Liter): Malat (5,0 g); K₂HPO₄ (0,5 g); MgSO₄x7H₂O (0,2 g); NaCl (0,1 g); CaCl₂x2H₂O (0,02 g); Bromthymolblau 0,5% in KOH 0,2 N (2 ml), sterilgefilterte Vitaminlösung (1 ml); sterilgefilterte Mikronährlösung (2 ml); 1,64% FeEDTA-Lösung (4 ml); KOH (4,5 g). Der pH wurde auf 6,5 eingestellt, und 1,8 g/l Agar wurden zugefügt. Die Mikronährlösung enthielt (Mengenangaben pro Liter): CuSO₄x5H₂O (40 mg); ZnSO₄x7H₂O (0,12 g); H₂BO₃ (1,4 g); Na₂MoO₄x2H₂O (1,0 g); MnSO₄xH₂O (1,175 g). JNFb enthielt zusätzliches K₂HPO₄ (0,6 g) und KH₂PO₄ (1,8 g), um den pH bei etwa 5,8 zu stabilisieren.

Nach 3 bis 5 Tagen Inkubation bei 30 °C wurden Pellikel-bildende Kulturen in frisches semisolides Medium übertragen. Die weitere Reinigung erfolgte auf NFb- (supplementiert mit 50 mg Hefeextrakt pro Liter) und ½ DYGS-Medium-Agarplatten (R. Neto et al. (1986), Suma Phytopathologica 12, 16, modifiziert). Die P-Kulturen wurden auf ½ DYGS-Medium aufbewahrt, das enthielt (Mengenangaben pro Liter): Glucose (1,0 g); Malat (1,0g); Hefeextrakt (2,0 g); Pepton (1,5 g); MgSO₄x7H₂O (0,5 g), L-Glutaminsäure (1,5 g). Der pH wurde auf 6,0 eingestellt.

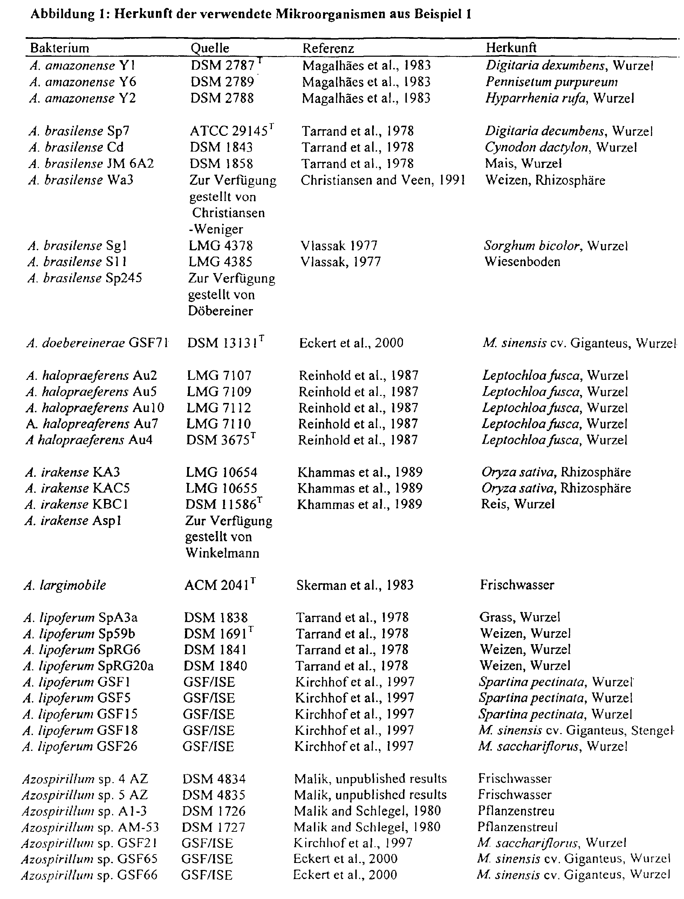

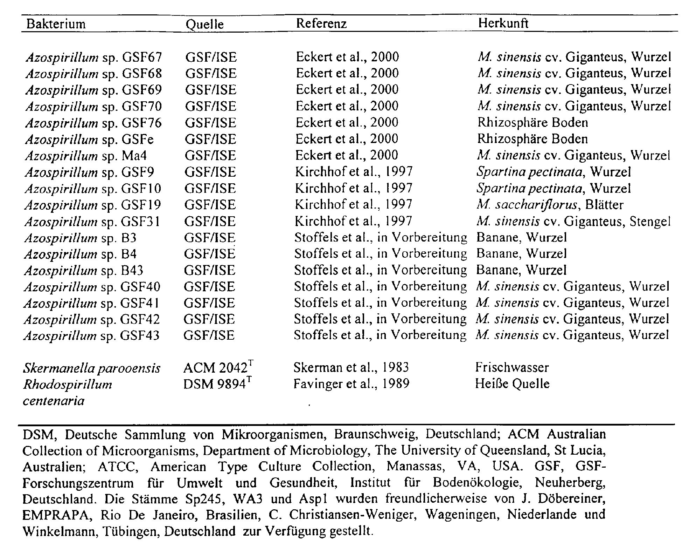

Sämtliche in Beispiel 1 verwendeten Stämme sind in Abbildung 1 aufgeführt.

### Oligonukleotidsonden:

Basierend auf der 16S-rDNA von *Azospirillum doebereinerae* (EMBL-Accession No. AJ238567) wurden die erfindungsgemäßen, für *Azospirillum* spezifischen Sonden Adoeb94 und Adoeb587 für die in situ-Hybridisierung mittels des PROBE DESIGN-Tool des Software-Pakets ARB (O. Strunk und W. Ludwig (1997), ARB: A Software environment for sequence data. [WWW-Dokument], URL http://www.mikro.biologie.tu-muenchen.de) entwickelt. Beide Sonden sind gegen die 16S-rRNA gerichtet. Die Sondensequenzen und exakten Targetpositionen sind in Abb. 2 angegeben.

Mit den Fluoreszenzfarbstoffen Cy3 und FLUOS markierte Oligonukleotidsonden wurden von INTERACTIVA (Ulm, Deutschland) bezogen.

### Zellfixierung:

Die Zellen aus exponentiell gewachsenen Kulturen wurden mit 4% (w/v) Paraformaldehyd gemäß Amann et al. (J. Bact. 172, 762-770 (1990)) fixiert. Die Zellsuspension wurde auf Objektträgern mittels Lufttrocknung immobilisiert. Die Zellen wurden durch Eintauchen der Platten in 50, 80 und 96% Ethanol weiterfixiert und dehydriert (R. I. Amann et al. (1995), Microbiol. Rev. 59, 143-169).

### In situ-Hybridisierung und Mikroskopie:

Die in situ-Ganzzell-Hybridisierung wurde durchgeführt, wie von Snaidr et al. (Appl. Environ. Microbiol. 63, 2884-2896 (1997)) beschrieben. Die Platten wurden mit einem Axioplan-Mikroskop (Zeiss, Oberkochen, Deutschland) unter Verwendung der Filtersets Nr. 09 und 15 untersucht. Zusätzlich wurde auch ein konfokales Laser-Scanning-Mikroskop (LSM 510, Zeiss, Oberkochen, Deutschland) verwendet.

### Einstellen stringenter Hybridisierungsbedingungen für die neu entwickelten Oligonukleotidsonden:

Die Stringenz der in situ-Hybridisierungsbedingungen für die erfindungsgemäßen Oligonukleotidsonden wurden durch schrittweises Erhöhen der Formamid-Konzentration in der Hybridisierungslösung erhöht (B. Manz et al. (1992), System Appl. Microbiol. 15, 593-600). Die in Abb. 2 als optimal empfohlenen Formamid-Konzentrationen sind die höchsten Konzentrationen, bei denen noch gute Signale mit den Zielzellen erhalten wurden und die zusätzlich eine Diskriminierung von Nicht-Targetzellen erlaubten.

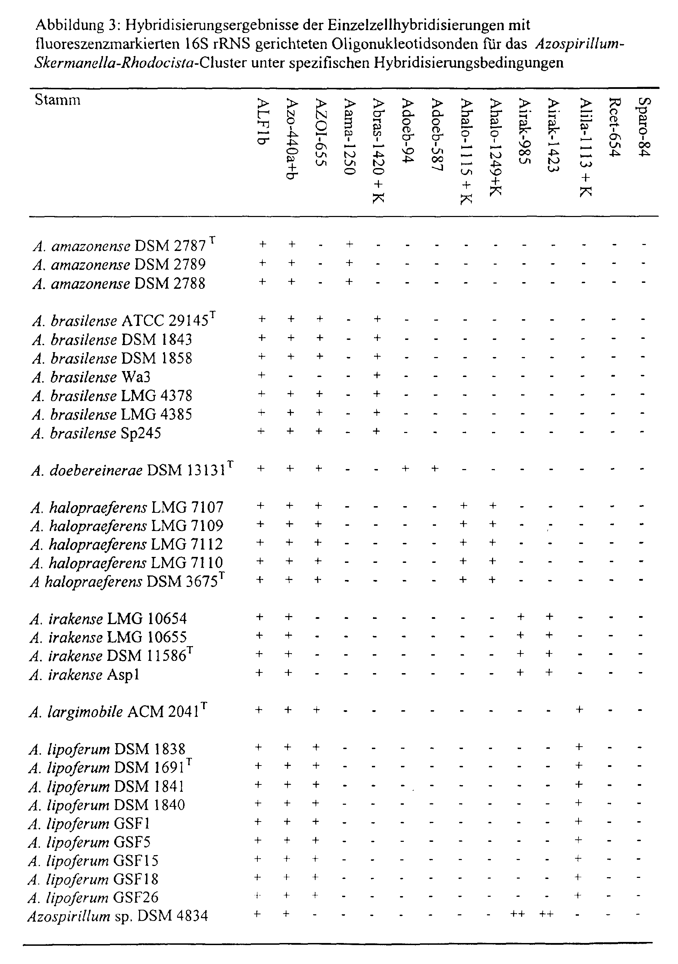

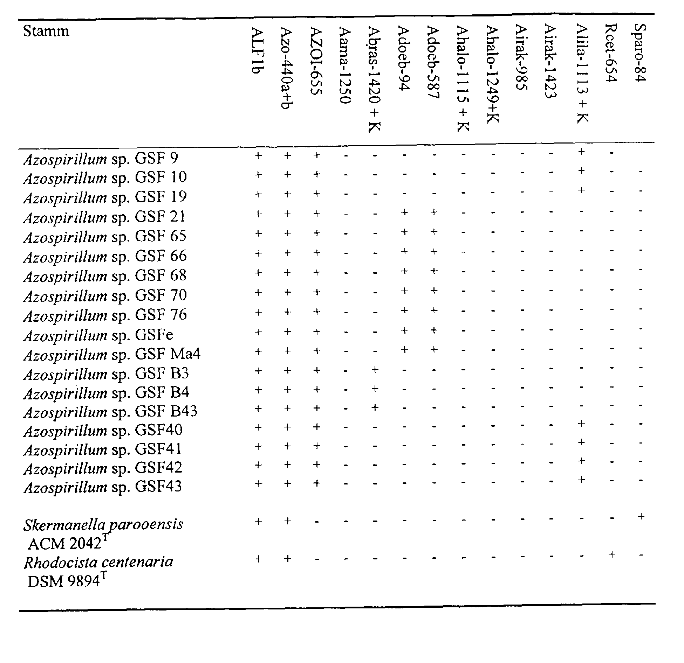

Die optimalen Hybridisierungsbedingungen wurden durch in situ-Ganzzellen-Hybridisierung von Referenzzellen mit fluoreszenzmarkierten Oligonukleotidsonden bewertet. Das erfindungsgemäße Oligonukleotid Adoeb587 hat nur einen einzigen destabilisierenden C-A-Mismatch an der Sondenposition 6 (5'-3') in den Targetsequenzen der zwei *A*. *lipoferum*-Stämme (DSM 1840 und DSM 1841) und *A*. *largimobile.* Die Bindung der Sonde Adoeb587 an die 16S-rDNA dieser Bakterien konnte durch die Zugabe von 30% Formamid zu der Hybridisierungslösung vollständig verhindert werden. Die Oligonukleotidsonde Adoeb94 hat mindestens zwei Mismatches zu allen anderen bekannten 16S- und 23S-rDNA-Sequenzen und zeigt ebenfalls helle und spezifische Hybridisierungssignale mit einer Formamid-Konzentration von 30% in der Hybridisierungslösung. Die zwei erfindungsgemäßen Sonden, zusammen mit der Sonde EUB338, die für alle Eubakteria spezifisch ist (Amann et al., supra), wurden erfolgreich für eine schnelle in situ-Ganzzellcharakterisierung von stickstoffixierenden Isolaten verwendet (siehe Abb. 3).

Die kombinierte Anwendung beider erfindungsgemäßer Sonden bietet die Möglichkeit zur in situ-Identifizierung und -Lokalisierung von *A. doebereinerae* in Umweltproben oder auf/in Pflanzengeweben und -extrakten, sowie ein schnelles und zuverlässiges Screenen von neuen Isolaten und Anreicherungskulturen. Die kombinierte Anwendung beider erfindungsgemäßer Sonden mit derselben Spezifität stellt eine zuverlässige Identifizierung der Spezies durch doppelte Anfärbung sicher. Zusätzlich können auch die anderen in den Tabellen aufgeführten Sonden verwendet werden, wobei auch die Hybridierung mit mehr als 2 Sonden möglich ist.

### Beispiel 2: Spezifische Oligonukleotidsonden für die Gattung Herbaspirillum

### Isolierung der Herbaspirillum-Stämme:

Gewaschene Wurzeln sowie luftausgesetzte Teile (Stengel, Blätter) von in Deutschland gezüchteten *Miscanthus sinensis* cv. Giganteus, *Miscanthus sacchariflorus* und *Spartina pectinata* sowie von in Brasilien gezüchteten *Pennisetum purpureum* cv. Cameroon, Cana d'Africa, Gramafante, Guaçu, Merker, Merkerx239, Mineiro, Mott, Piracicaba, Roxo, s/pelo und Taiwan wurden aufgeweicht. Eine Reihe von Verdünnungen in 4%iger Saccharose-Lösung bis zu einer Verdünnung von 10⁻⁶ wurde in NFb- oder JNFb-semisolide Medien (Döbereiner (1995), supra) enthaltende Röhrchen inokuliert.

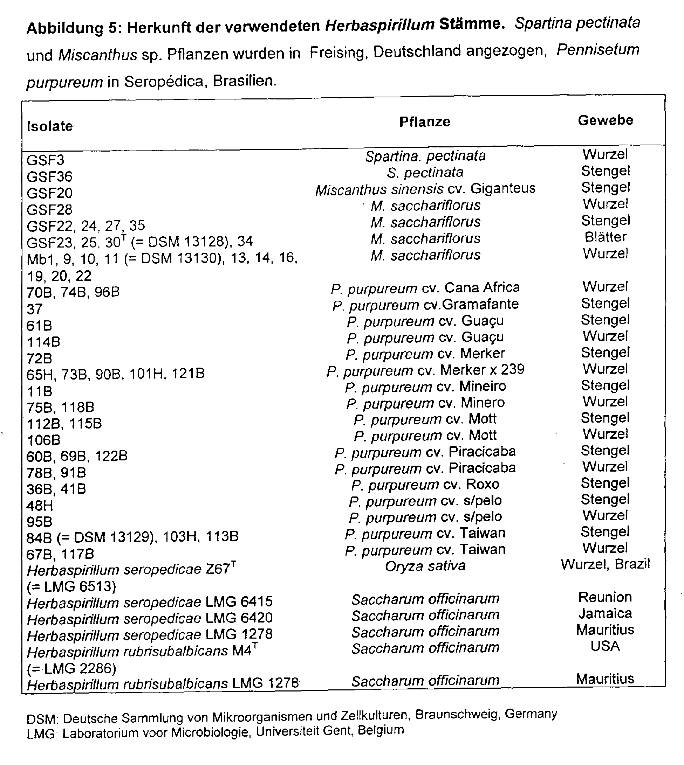

Nach 4 bis 6 Tagen Inkubation bei 30 °C wurden die Pellikel-bildenden Bakterien einer weiteren Reinigung durch Ausstreichen auf NFb- und JNFb-Agarplatten, die zusätzlich 20 mg/l Hefeextrakt enthielten, unterzogen, und die Einzelkolonien wurde wiederum in einem geeigneten semisoliden Medium kultiviert. Die Herkunft der Stämme und die Quellen des Pflanzengewebes sind in Abbildung 5 aufgeführt.

Basierend auf dem PROBE DESIGN-Tool des Software-Pakets ARB (Strunk & Ludwig, supra) wurden die 16S-rDNA-Sequenzen auf diagnostische Regionen in der Basenzusammensetzung für die Gattung *Herbaspirillum* und die Arten *H*. *rubrisubalbicans*, *H*. *seropedicae* und *H*. *frisingense* untersucht. Es konnten zwei erfindungsgemäße gattungsspezifische (Herb 68, Herb 1432) sowie drei erfindungsgemäße artspezifische Oligonukleotidsonden für die Arten *H. rubrisubalbicans*, *H. seropedicae* sowie *H. frisingense* entwickelt und für in situ-Hybridisierungsexperimente optimiert werden (Amann et al., supra; siehe Abb. 2).

Die Spezifität und Empfindlichkeit der erfindungsgemäßen Sonden wurde durch Hybridisierung mit Mischungen von Paraformaldehyd-fixierten Zellen von *Herbaspirilla* und Referenzorganismen von phylogenetisch verwandten Taxa bewertet. Stringente Hybridisierungsbedingungen wurden durch schrittweises Erhöhen der Formamid-Konzentration im Hybridisierungspuffer unter Beibehaltung der Ionenstärken (0,9 M Natriumchlorid) und der Hybridisierungstemperatur (46°C) eingestellt.

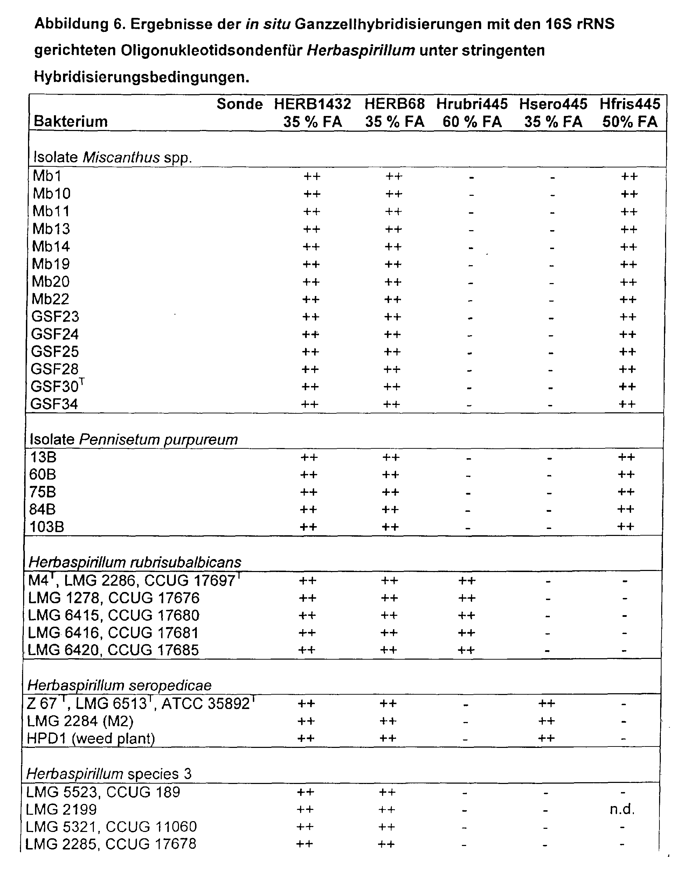

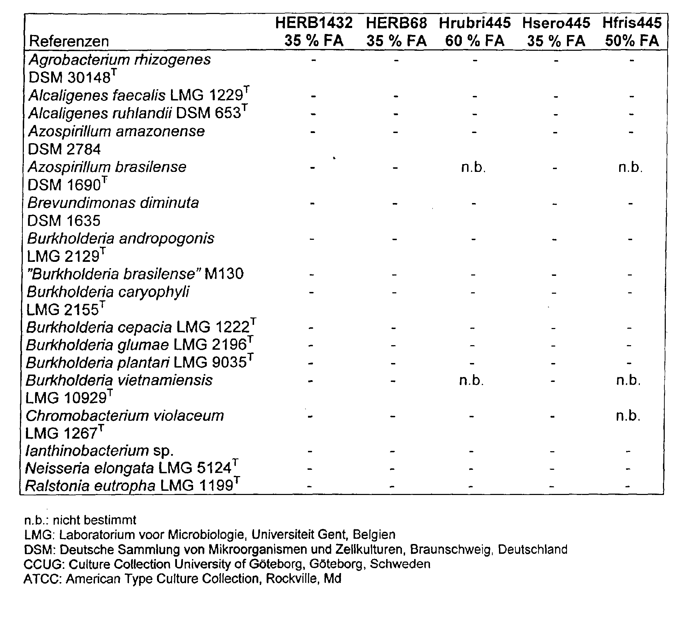

Die zwei gattungsspezifischen Sonden Herb 68 und Herb 1432 sind komplementär zu verschiedenen Bereichen der 16S-rRNA und hybridisieren unter stringenten Hybridisierungsbedingungen mit allen Mitgliedern der Gattung *Herbaspirillum* (siehe Abb. 6). Die artspezifischen Sonden Hsero 443, Hrubri 443 und Hfris 443 sind auf den selben diagnostischen Bereich innerhalb der 16S-rRNA gerichtet (Position in *E.coli* 445-462). Sie können als diagnostische Tools zur Unterscheidung von *H. rubrisubalbicans*, *H. seropedicae* und *H*. *frisingense* auf Artebene verwendet werden. Die Anwendung des erfindungsgemäßen *Herbaspirillum-Sondensets* erlaubte eine schnelle und hochzuverlässige Identifizierung der Referenzstämme und -isolate, die zu dieser Gattung gehören, sowie eine direkte Identifizierung/Lokalisierung von entsprechenden Bakterien in/auf Pflanzengeweben und -extrakten.

### Abbildungen

Abbildung 1: Herkunft der verwendeten Mikroorganismen aus Beispiel 1.

Abbildung 2: Art- und/oder gattungsspezifische rRNA-gerichtete Oligonukleotidsonden für Bakterien des Gattungsclusters *Azospirillum*, *Skermanella*, *Rhodocista*, sowie der Gattung *Herbaspirillum* und für *Acetobacter diazotrophicus*, ihre Sequenzen, Targetstellen, Spezifitäten und Formamid-Konzentrationen, die im Hybridisierungspuffer für spezifische in situ-Hybridisierungen geeignet sind.

Abbildung 3: Ergebnisse der in situ-Ganzzellhybridisierungen mit den 16S-rRNA-gerichteten Oligonukleotidsonden für das *Azospirillum-Rhodocista-Skermanella-Cluster* unter stringenten Hybridisierungsbedingungen.

Abbildung 4: Stammbaum, der die phylogenetische Verwandtschaft der Gattungen und Arten innerhalb des *Azospirillum-Rhodocista-Skermanella-Clusters* basierend auf vergleichender 16S-rRNA-Sequenzanalyse wiedergibt. Der Baum zeigt eine Zusammenfassung der neu entwickelten 16S-rRNA gerichteten Oligonukleotidsonden für dieses Cluster. Die Spezifität der Sonden ist durch Klammern dargestellt.

Abbildung 5: Herkunft der *Herbaspirillum*-Stämme aus Beispiel 2.

Abbildung 6: Ergebnisse der in situ-Ganzzellhybridisierungen mit den 16S-rRNA-gerichteten Oligonukleotidsonden für *Herbaspirillum* unter stringenten Hybridisierungsbedingungen.

Sequenzprotokoll (entsprechend der Numerierung in Abbildung 2):

SEQ ID No. 1: 5'-GTCATCATCGTCGCGTGC-3'

SEQ ID No. 2: 5'-GTCATCATCGTCGTGTGC-3'

SEQ ID No. 3: 5'-CACCATCCTCTCCGGAAC-3'

SEQ ID No. 4:	5'-CACGAGGTCGCTGCCCAC-3'

SEQ ID No. 5:	5'-CCACCTTCGGGTAAAGCCA-3'

SEQ ID No. 6: 5'-ACTTCCGACTAAACAGGC-3'

SEQ ID No. 7: 5'-CGTGCGCCACTGTGCCGA-3'

SEQ ID No. 8:	5'-ATGGTGGCAACTGGCAGCA-3'

SEQ ID No. 9: 5'-GCGACGTCGCTTCCCACT-3'

SEQ ID No. 10: 5'-CACCGGCTCAGGTAAAG-3'

SEQ ID No. 11:	5'-TCAAGGCATGCAAGGGTT-3'

SEQ ID No. 12: 5'-ATGGCAACTGACGGTAGG-3'

SEQ ID No. 13: 5'-ACCCACCTCTCCGGACCT-3'

SEQ ID No. 14: 5'-CGTGCGCCACTAGGGGCG -3'

SEQ ID No. 15: 5'-ATGATGGCAACTGGCAGTA-3'

SEQ ID No. 16: 5'-GCGACTTCGCTTCCCACT-3'

SEQ ID No. 17: 5'-CACCTTCGGGTAAAACCA-3'

SEQ ID No. 18: 5'-ATGGCAACTGGCGGTAGG-3'

SEQ ID No. 19: 5'-AGCAAGCTCCTATGCTGC-3'

SEQ ID No. 20: 5'-CGGTTAGGCTACCCACTT-3'

SEQ ID No. 21: 5'-GCCAAAACCGTTTCTTCC-3'

SEQ ID No. 22: 5'-GCTACCACCGTTTCTTCG-3'

SEQ ID No. 23:	5'-TCCAGAACCGTTTCTTCC-3'

SEQ ID No. 24:	5'-GGCTGCGCCCCATTGCTG-3'

## Patentansprüche

1. Oligonukleotidsonden, die spezifisch an die 16S-rRNA von Bakterienzellen des Gattungsc lusters *Azospirillum*, *Skermanella*, *Rhodocista* hybridisieren, ausgewählt aus einer Gruppe bestehend aus:
a) DNA-Sequenzen mit den in SEQ ID No. 1 bis 18 angegebenen Nukleotidsequenzen;
b) DNA-Sequenzen, umfassend eine Nukleotidsequenz, die sich von den in SEQ ID No. 1 bis 18 angegebenen Nukleotidsequenzen um bis zu 3 Basen unterscheidet; und
c) DNA-Sequenzen, die die Sequenzen a) und b) oder Fragmente davon umfassen.

2. Oligonukleotidsonden, die spezifisch an die 16S-rRNA von Bakterienzellen der Gattung *Herbaspirillum* hybridisieren, ausgewählt aus einer Gruppe bestehend aus:
a) DNA-Sequenzen mit den in SEQ ID No. 19 bis 23 angegebenen Nukleotidsequenzen;
b) DNA-Sequenzen, umfassend eine Nukleotidsequenz, die sich von den in SEQ ID No. 19 bis 23 angegebenen Nukleotidsequenzen um bis zu 3 Basen unterscheidet; und
c) DNA-Sequenzen, die die Sequenzen a) und b) oder Fragmente davon umfassen.

3. Oligonukleotidsonden, die spezifisch an die 16S-rRNA von Bakterienzellen der Art *Acetobacter diazotrophicus* hybridisieren, ausgewählt aus einer Gruppe bestehend aus:
a) DNA-Sequenz mit der in SEQ ID No. 24 angegebenen Nukleotidsequenz;
b) DNA-Sequenzen, umfassend eine Nukleotidsequenz, die sich von der in SEQ ID No. 24 angegebenen Nukleotidsequenz um bis zu 3 Basen unterscheidet; und
c) DNA-Sequenzen, die die Sequenzen a) und b) oder Fragmente davon umfassen.

4. Verfahren zum art- und/oder gattungsspezifischen Nachweis von PGPR-Bakterien (plant growth promoting bacteria) durch in situ-Einzelzellhybridisierung, umfassend die folgenden Schritte:
d) Fixieren der PGPR-Bakterien;
e) In situ-Inkubation der fixierten Bakterien mit Oligonukleotidsonden, die spezifisch an die 16S-rRNA von PGPR-Bakterienzellen hybridisieren;
f) Detektieren und gegebenenfalls Quantifizieren der PGPR-Bakterien.

5. Verfahren nach Anspruch 4, wobei in Schritt b) Kompetitor-Oligonukleotidsonden zur Erhöhung der Spezifität eingesetzt werden.

6. Verfahren nach Anspruch 4 oder 5, wobei der Nachweis in situ in Pflanzenmaterial, Reinkulturen und/oder Anreicherungskulturen erfolgt.

7. Verfahren nach einem der Ansprüche 4 bis 6, wobei die Detektion der Oligonukleotidsonden über an die Oligonukleotidsonden gekoppelte detektierbare Marker oder über mit detektierbaren Markern gekoppelte Antikörper erfolgt.

8. Verfahren nach einem der Ansprüche 4 bis 7, wobei der detektierbare Marker aus der Gruppe, bestehend aus Fluoreszenzmarkern, Chemolumineszenzmarkern, radioaktiven Markern und enzymatischen Gruppen, ausgewählt wird.

9. Verfahren nach einem der Ansprüche 4 bis 8, wobei als Oligonukleotidsonden bzw. als Kompetitor-Oligonukleotidsonden die Oligonukleotidsonden nach einem der Ansprüche 1 bis 3 verwendet werden.

10. Kit, umfassend mindestens eine Oligonukleotidsonde nach einem der Ansprüche 1 bis 3.

11. Verwendung einer Oligonukleotidsonde oder eines Nukleinsäuremoleküls nach einem der Ansprüche 1 bis 3 zum art- und/oder gattungsspezifischen Nachweis von PGPR-Bakterien, ausgewählt aus dem Gattungscluster *Azospirillum*, *Skermanella*, *Rhodocista*, der Gattung *Herbaspirillum* und der Art *Acetobacter diazotrophicus.*

12. Verwendung nach Anspruch 11 zur Lokalisierung und Quantifizierung von PGPR-Bakterien in Pflanzenmaterialien.

13. Verwendung nach Anspruch 11 zur Identifizierung von Isolaten von PGPR-Bakterien.

14. Verwendung nach einem der Ansprüche 11 bis 13 in automatisierten Testverfahren für PGPR-Bakterien, insbesondere in auf Mikrochips durchgeführten Nachweisverfahren.

15. Verwendung nach einem der Ansprüche 11 bis 14, wobei die Oligonukleotidsonden als Hybridsierungssonden in Dot-Blot-Hybridisierungen eingesetzt werden.

16. Verwendung nach einem der Ansprüche 11 bis 14, wobei auf der Grundlage dieser Sonden konstruierte Oligonukleotidprimer bzw. Oligonukleotidprimer-Paare in einem auf Amplifizierung durch PCR basierenden Verfahren eingesetzt werden.

17. Verwendung nach einem der Ansprüche 11 bis 14, wobei die Oligonukleotidsonden in einem Verfahren gemäß einem der Ansprüche 4 bis 9 eingesetzt werden.


SEQUENZPROTOKOLL

<110> GSF - Forschungszentrum für Umwelt und Gesundheit

<120> Oligonukleotidsonden zum art- und/oder
      gattungsspezifischen Nachweis von das Pflanzenwachstum
      fördernden Bakterien

<130> G 7213

<140>
<141>

<150> DE 100 10 614.5
<151> 2000-03-03

<160> 24

<170> PatentIn Ver. 2.1

<210> 1
<211> 18
<212> DNA
<213> Künstliche Sequenz

<220>
<223> Beschreibung der künstlichen Sequenz:
      Oligonukleotidsonde

<400> 1
gtcatcatcg tcgcgtgc                                                18


<210> 2
<211> 18
<212> DNA
<213> Künstliche Sequenz

<220>
<223> Beschreibung der künstlichen Sequenz:
      Oligonukleotidsonde

<400> 2
gtcatcatcg tcgtgtgc                                                18


<210> 3
<211> 18

<212> DNA
<213> Künstliche Sequenz

<220>
<223> Beschreibung der künstlichen Sequenz:
      Oligonukleotidsonde

<400> 3
caccatcctc tccggaac                                                    18


<210> 4
<211> 18
<212> DNA
<213> Künstliche Sequenz

<220>
<223> Beschreibung der künstlichen Sequenz:
      Oligonukleotidsonde

<400> 4
cacgaggtcg ctgcccac                                                    18


<210> 5
<211> 19
<212> DNA
<213> Künstliche Sequenz

<220>
<223> Beschreibung der künstlichen Sequenz:
      Oligonukleotidsonde

<400> 5
ccaccttcgg gtaaagcca                                                   19


<210> 6
<211> 18
<212> DNA
<213> Künstliche Sequenz

<220>
<223> Beschreibung der künstlichen Sequenz:
      Oligonukleotidsonde

<400> 6
acttccgact aaacaggc                                                    18

<210> 7
<211> 18
<212> DNA
<213> Künstliche Sequenz

<220>
<223> Beschreibung der künstlichen Sequenz:
      Oligonukleotidsonde

<400> 7
cgtgcgccac tgtgccga                                                    18


<210> 8
<211> 19
<212> DNA
<213> Künstliche Sequenz

<220>
<223> Beschreibung der künstlichen Sequenz:
      Oligonukleotidsonde

<400> 8
atggtggcaa ctggcagca                                                   19


<210> 9
<211> 18
<212> DNA
<213> Künstliche Sequenz

<220>
<223> Beschreibung der künstlichen Sequenz:
      Oligonukleotidsonde

<400> 9
gcgacgtcgc ttcccact                                                    18


<210> 10
<211> 17
<212> DNA
<213> Künstliche Sequenz

<220>
<223> Beschreibung der künstlichen Sequenz:

Oligonukleotidsonde

<400> 10
caccggctca ggtaaag                                                17

<210> 11
<211> 18
<212> DNA
<213> Künstliche Sequenz

<220>
<223> Beschreibung der künstlichen Sequenz:
      Oligonukleotidsonde

<400> 11
tcaaggcatg caagggtt                                               18

<210> 12
<211> 18
<212> DNA
<213> Künstliche Sequenz

<220>
<223> Beschreibung der künstlichen Sequenz:
      Oligonukleotidsonde

<400> 12
atggcaactg acggtagg                                               18

<210> 13
<211> 18
<212> DNA
<213> Künstliche Sequenz

<220>
<223> Beschreibung der künstlichen Sequenz:
      Oligonukleotidsonde

<400> 13
acccacctct ccggacct                                               18

<210> 14
<211> 18
<212> DNA

<213> Künstliche Sequenz

<220>
<223> Beschreibung der künstlichen Sequenz:
      Oligonukleotidsonde

<400> 14
cgtgcgccac taggggcg                                                    18


<210> 15
<211> 19
<212> DNA
<213> Künstliche Sequenz

<220>
<223> Beschreibung der künstlichen Sequenz:
      Oligonukleotidsonde

<400> 15
atgatggcaa ctggcagta                                                   19


<210> 16
<211> 18
<212> DNA
<213> Künstliche Sequenz

<220>
<223> Beschreibung der künstlichen Sequenz:
      Oligonukleotidsonde

<400> 16
gcgacttcgc ttcccact                                                    18


<210> 17
<211> 18
<212> DNA
<213> Künstliche Sequenz

<220>
<223> Beschreibung der künstlichen Sequenz:
      Oligonukleotidsonde

<400> 17
caccttcggg taaaacca                                                    18

<210> 18
<211> 18
<212> DNA
<213> Künstliche Sequenz

<220>
<223> Beschreibung der künstlichen Sequenz:
      Oligonukleotidsonde

<400> 18
atggcaactg gcggtagg                                                    18


<210> 19
<211> 18
<212> DNA
<213> Künstliche Sequenz

<220>
<223> Beschreibung der künstlichen Sequenz:
      Oligonukleotidsonde

<400> 19
agcaagctcc tatgctgc                                                    18


<210> 20
<211> 18
<212> DNA
<213> Künstliche Sequenz

<220>
<223> Beschreibung der künstlichen Sequenz:
      Oligonukleotidsonde

<400> 20
cggttaggct acccactt                                                    18


<210> 21
<211> 18
<212> DNA
<213> Künstliche Sequenz

<220>
<223> Beschreibung der künstlichen Sequenz:
      Oligonukleotidsonde

<400> 21
gccaaaaccg tttcttcc                                                    18

<210> 22
<211> 18
<212> DNA
<213> Künstliche Sequenz

<220>
<223> Beschreibung der künstlichen Sequenz:
      Oligonukleotidsonde

<400> 22
gctaccaccg tttcttcg                                                    18

<210> 23
<211> 18
<212> DNA
<213> Künstliche Sequenz

<220>
<223> Beschreibung der künstlichen Sequenz:
      Oligonukleotidsonde

<400> 23
tccagaaccg tttcttcc                                                    18

<210> 24
<211> 18
<212> DNA
<213> Künstliche Sequenz

<220>
<223> Beschreibung der künstlichen Sequenz:
      Oligonukleotidsonde

<400> 24
ggctgcgccc cattgctg                                                    18